# EUROPEAN PATENT APPLICATION

(11) **EP 3 124 469 A1**
(43) Date of publication of application: **01.02.2017**
(21) Application number: 15768994.4
(22) Date of filing: 23.03.2015
(51) Int. Cl.: C07C 251/16, C07C 227/18, C07C 227/32, C07C 229/48, C07C 249/02, C07B 53/00, C07B 61/00

(54) **PROCESS FOR PRODUCING 1-ARYLIMINO-2-VINYLCYCLOPROPANECARBOXYLIC ACID DERIVATIVE**

(30) Priority: 28.03.2014 JP 2014068837
(71) Applicant: Kaneka Corporation, Osaka-shi, Osaka 530-8288 (JP)
(72) Inventor: MURATA, Takahiko, Takasago-shi Hyogo 676-8688 (JP); FUNABASHI, Makoto, Takasago-shi Hyogo 676-8688 (JP); NISHIYAMA, Akira, Takasago-shi Hyogo 676-8688 (JP)
(74) Representative: Vossius & Partner Patentanwälte Rechtsanwälte mbB
(86) International application number: PCT/JP2015/058671
(87) International publication number: WO 2015/146881

(57) **Abstract**

The present invention relates to a method of producing the 1-arylimino-2-vinylcyclopropanecarboxylic acid derivative represented by the formula (2) by reacting an N-(arylmethylene)glycine ester represented by the formula (1) with (E)-1,4-dibromo-2-butene using a sodium ethoxide at a yield which is close to that which is achieved when lithium tert-butoxide is used, preferably at a yield which is higher than or equal to that which is achieved when lithium tert-butoxide is used, without posing a problem with industrial-scale production.

Further, the present invention contains a method of producing a (1R,2S)-1-arylimino-2-vinylcyclopropanecarboxylic acid derivative represented by the formula (4) by reacting an N-(arylmethylene)glycine ester represented by the formula (1) with (E)-1,4-dibromo-2-butene using a sodium ethoxide in the presence of an optically active catalyst.

## Description

### TECHNICAL FIELD

The present invention relates to a method of producing a 1-arylimino-2-vinylcyclopropanecarboxylic acid derivative which is useful as an intermediate for pharmaceuticals, particularly a therapeutic agent for hepatitis C.

### BACKGROUND ART

(1R,2S)-1-amino-2-vinylcyclopropanecarboxylic acid derivative is useful as an intermediate for a therapeutic agent for hepatitis C, and a method of producing it is discussed in detail in Non-Patent Literature 1. In Non-Patent Literature 1, N-(phenylmethylene)glycine ethyl ester, indicated by compound No. 4, is reacted with (E)-1,4-dibromo-2-butene at room temperature in the present of a base, to produce 1-phenylimino-2-vinylcyclopropanecarboxylic acid ethyl ester, indicated by compound No. 6 below (see a formula below hereinafter referred to as "cyclopropanation reaction"), and then the imino group is hydrolyzed, to produce a 1-amino-2-vinylcyclopropanecarboxylic acid derivative.

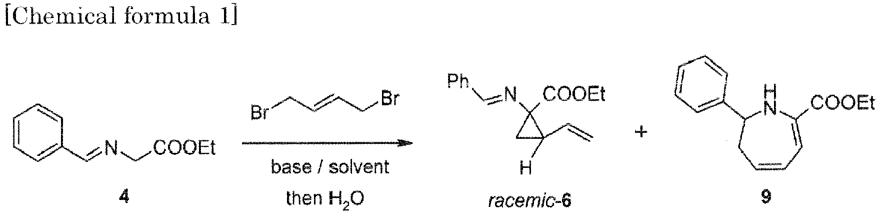

In this cyclopropanation reaction, compound No. 9 is formed as a by-product. It is considered that the by-product 9 is produced via intermediate formation of a cis imine (5) represented by a formula below. The cis imine is a diastereoisomer of compound No. 6, which is a target compound.

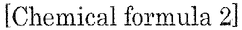

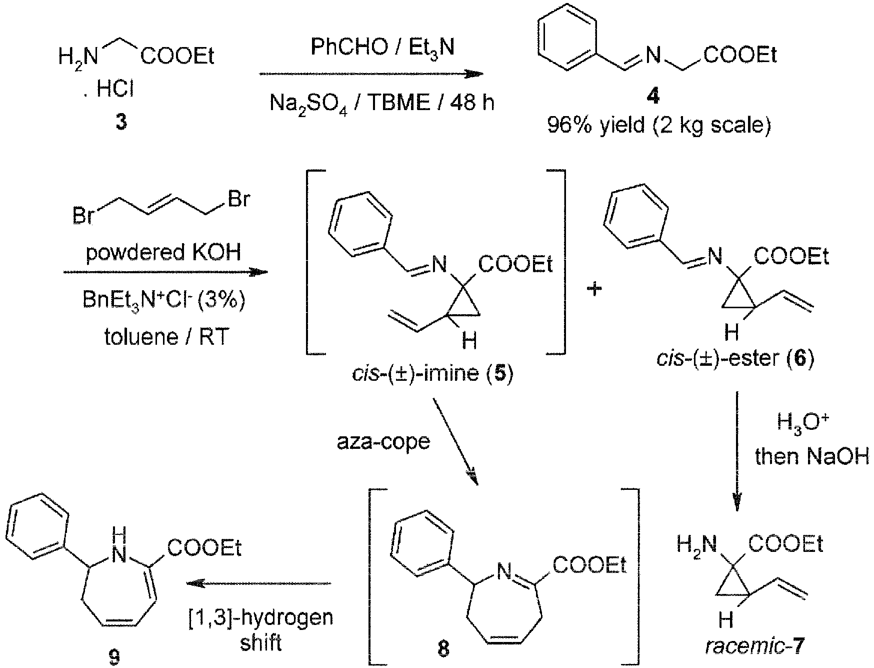

Non-Patent Literature 1 indicates that, in order to increase the diastereoselectivity of the target cis ester (6) rather than the cis imine (5) which causes the production of the by-product 9, it is effective to provide an intermediate having minimal steric interactions as shown by a formula below, and an optimum way to do so is to use a (hindered) tert-butoxy base which causes high steric hindrance. Further, Non-Patent Literature 1 indicates that the reaction temperature does not have an influence on the result, that a nonpolar solvent, such as toluene or the like, is more desirable than a polar solvent, such as THF or the like, and that, of the tert-butoxy bases, the Li salt, the Na salt, and the K salt (M⁺ in the formula below), which have increasing basicity, show decreasing diastereoselectivity, and of the hexamethyldisilazane bases, the Li salt, Na salt, and the K salt, which have increasing basicity, show decreasing diastereoselectivity. Therefore, it is considered that it is most appropriate to use lithium tert-butoxide in toluene.

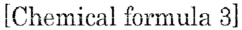

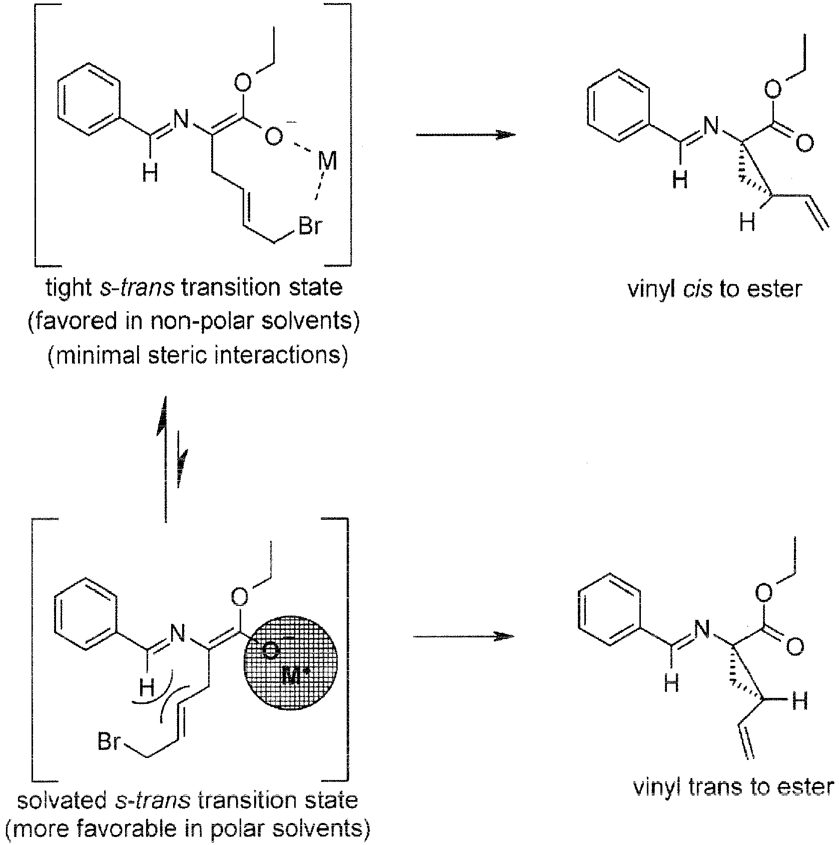

The matter discussed above is employed in Patent Literature 1 and Patent Literature 2. In Patent Literature 1 and Patent Literature 2, the reaction between N-(phenylmethylene)glycine ethyl ester and (E)-1,4-dibromo-2-butene to produce 1-phenylimino-2-vinylcyclopropanecarboxylic acid ethyl ester is conducted using lithium tert-butoxide in toluene.

In each of Patent Literature 1 and Patent Literature 2, products (1-phenylimino-2-vinylcyclopropanecarboxylic acid derivatives) by the cyclopropanation reaction are all a racemate. An optically active 1-amino-2-vinylcyclocarboxylic acid derivative is eventually obtained by asymmetric hydrolysis using Alcalase in Non-Patent Literature 1, by salt formation/crystallization using an optically active acid in Patent Literature 1, and by asymmetric hydrolysis using a hydrase in Patent Literature 2.

### PRIOR ART DOCUMENT

### PATENT DOCUMENT

Patent Literature 1: Japanese Unexamined Patent Publication No. 2011-46613A
Patent Literature 2: WO2011/003063

### NON-PATENT DOCUMENT

Non-Patent Literature 1: J. Org. Chem, 2005, 70(15), 5869-5879.

### SUMMARY OF THE INVENTION

### PROBLEMS TO BE SOLVED BY THE INVENTION

However, it is difficult to obtain lithium tert-butoxide, which is considered to be most suitable for the cyclopropanation reaction, at low cost, in a short lead time, and in a stable manner, which poses a problem with industrial-scale production. Further, the present inventors have found that, in the cyclopropanation reaction employing lithium tert-butoxide, only a racemate is obtained even using an asymmetric catalyst, and an optically active cyclopropanated product cannot be produced.

Therefore, it is an object of the present invention to provide a method of producing a 1-arylimino-2-vinylcyclopropanecarboxylic acid derivative at a yield which is close to that which is achieved when lithium tert-butoxide is used, preferably at a yield which is higher than or equal to that which is achieved when lithium tert-butoxide is used, without posing a problem with industrial-scale production, or a method of directly producing an optically active 1-arylimino-2-vinylcyclopropanecarboxylic acid derivative by the cyclopropanation reaction.

### SOLUTIONS TO THE PROBLEMS

The present inventors have thoroughly studied in order to solve the above problems. As a result, the present inventors have found that, of the alkoxy groups having low steric hindrance, which are traditionally considered to be unfavorable, only an ethoxy group provides a yield which is higher than or equal to that which is achieved when lithium tert-butoxide is used, when it is combined with sodium, which is traditionally considered to be unfavorable. This is unexpected and the opposite of these traditional suggestion. Further, the present inventors have eventually found that when sodium ethoxide is used as a base, an optically active 1-arylimino-2-vinylcyclopropanecarboxylic acid derivative can be produced using an asymmetric catalyst, and sodium ethoxide thus has a particularly superior effect. Accordingly, the present invention has been made.

That is, the present invention is a method of producing a 1-arylimino-2-vinylcyclopropanecarboxylic acid derivative, comprising
reacting an N-(arylmethylene)glycine ester represented by the following formula (1): 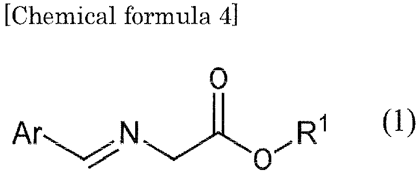 wherein Ar represents a C₆-C₁₂ aryl group and R¹ represents a C₁-C₆ alkyl group, with (E)-1,4-dibromo-2-butene using a sodium ethoxide, to produce the 1-aryllimino-2-vinylcyclopropanecarboxylic acid derivative represented by the following formula (2): 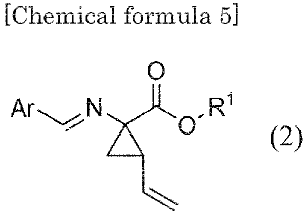 wherein Ar and R¹ are the same as described above.

Further, the present invention also contains a method of producing a (1R,2S)-1-arylimino-2-vinylcyclopropanecarboxylic acid derivative, comprising
reacting an N-(arylmethylene)glycine ester represented by the following formula (1): 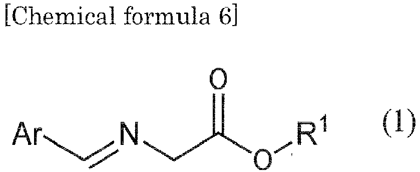 wherein Ar represents a C₆-C1₂ aryl group, and R¹ represents a C₁-C₆alkyl group, with (E)-1,4-dibromo-2-butene using a sodium ethoxide in the presence of an optically active catalyst, to produce the (1R,2S)-1-arylimino-2-vinylcyclopropanecarboxylic acid derivative represented by the following formula (4): 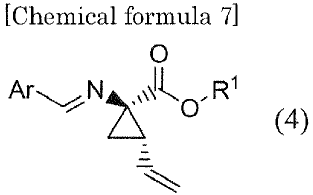 wherein Ar and R¹ are the same as described above.

### EFFECTS OF THE INVENTION

According to the present invention, an N-(arylmethylene)glycine ester is reacted with (E)-1,4-dibromo-2-butene using sodium ethoxide, and therefore, a (1R,2S)-1-amino-2-vinylcyclopropanecarboxylic acid derivative, which is important for production of pharmaceuticals and the like, can be produced on an industrial scale, at low cost, and in a stable manner.

### MODE FOR CARRYING OUT THE INVENTION

The present invention will now be described in detail.

Firstly, in the present invention, an N-(arylmethylene)glycine ester (1) represented by the following formula (1): 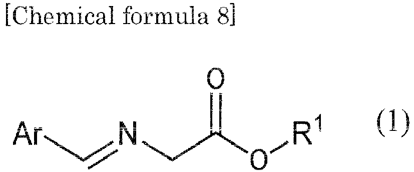 is reacted with (E)-1,4-dibromo-2-butene using sodium ethoxide, to produce a 1-arylimino-2-vinylcyclopropanecarboxylic acid derivative (2) represented by the following formula (2) : 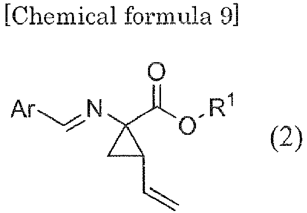 wherein Ar is a C₆-C₁₂ aryl group. Specifically, for example, Ar is a phenyl group, a 1-naphtyl group, a 2-naphtyl group, a p-methylphenyl group, an o-chlorophenyl group, an m-chlorophenyl group, a p-chlorophenyl group, a p-bromophenyl group, a p-fluorophenyl group, a p-trifluoromethyl group, a p-nitrophenyl group, an o-methoxyphenyl group, an m-methoxyphenyl group, or p-methoxyphenyl group. Ar is preferably a phenyl group, a p-chlorophenyl group, or a p-methoxyphenyl group, more preferably a phenyl group.

Also, R¹ represents a C₁-C₆ alkyl group. Specifically, for example, R¹ is a methyl group, an ethyl group, an n-propyl group, an isopropyl group, an n-butyl group, an isobutyl group, a sec-butyl group, or a tert-butyl group, preferably a methyl group, an ethyl group, or a tert-butyl group, and more preferably an ethyl group. The compound (1) can be produced according to a known method (e.g., J. Org. Chem, 2005, 70(15), 5869-5879.). Alternatively, a commercially available product can be used. Specifically, the compound (1) can be produced by reacting H₂N-CH₂-COOR¹ (R¹ is the same as described above) or a salt thereof with Ar-CHO in the presence of a base such as an alkylamine or the like.

The amount of the compound (1) to be used is 0.5-20 equivalents, more preferably 0.8-10 equivalents, and particularly preferably 1-5 equivalents, with respect to (E)-1.4-dibromo-2-butene, because the excessive amount is undesirable from a cost standpoint.

The form of the above sodium ethoxide is not particularly limited, and may be any form, comprising powder, solution obtained by dissolution in a solvent such as ethanol or the like, suspension, or the like. The amount of sodium ethoxide to be used is 1-20 equivalents, more preferably 1.3-10 equivalents, and particularly preferably 1.5-5 equivalents, with respect to (E)-1,4-dibromo-2-butene, because the excessive amount is undesirable from a cost standpoint.

A solvent for this reaction is not particularly limited as long as the solvent does not have an influence on the reaction. Specific examples of the solvent include: alcohol solvents such as methanol, ethanol, n-propanol, isopropanol, n-butanol, tert-butanol, ethylene glycol, and the like; ether solvents such as tetrahydrofuran, diethyl ether, 1,4-dioxane, methyl tert-butyl ether, ethylene glycol dimethyl ether, and the like; nitrile solvents such as acetonitrile, propionitrile, and the like; ester solvents such as ethyl acetate, n-propyl acetate, isopropyl acetate, and the like; aliphatic hydrocarbon solvents such as pentane, hexane, heptane, methylcyclohexane, and the like; aromatic hydrocarbon solvents such as benzene, toluene, xylene, ethylbenzene, mesitylene, and the like; ketone solvents such as acetone, methyl ethyl ketone, and the like; halogen solvents such as methylene chloride, 1,2-dichloroethane, chlorobenzene, and the like; sulfoxide solvents such as dimethyl sulfoxide and the like; amide solvents such as N,N-dimethylformamide, N,N-dimethylacetamide, N,N-diethylacetamide, N-methyl-2-pyrrolidone, N-ethyl-2-pyrrolidone, N-methyl-ε-caprolactam, hexamethylphosphoramide, and the like; urea solvents such as dimethylpropyleneurea and the like; phosphoric triamide solvents such as hexamethylphosphoric triamide and the like; and the like. When lithium tert-butoxide is used, then if toluene is replaced with a polar solvent such as THF or the like, the yield significantly decreases. In contrast, the method of the present invention in which sodium ethoxide is used can achieve a high reaction yield no matter whether a nonpolar solvent or a polar solvent is used. These solvents may be used alone or in combination. When two or more solvents are used in combination, the mixing ratio is not particularly limited. The solvent is preferably ethanol, methyl tert-butyl ether, hexane, heptane, toluene, xylene, ethylbenzene, mesitylene, methylene chloride, 1,2-dichloroethane, or chlorobenzene, more preferably methyl tert-butyl ether or toluene.

The upper limit of the amount of the solvent to be used is preferably 100 times, more preferably 50 times, and particularly preferably 20 times the weight of (E)-1,4-dibromo-2-butene, because the excessive amount is undesirable from a cost or post-treatment standpoint. The lower limit is preferably 0.1 times, more preferably 0.5 times, and particularly preferably one time the weight of (E)-1,4-dibromo-2-butene.

The reaction temperature of this reaction is not particularly limited. and may be set as appropriate. In order to reduce the formation of a by-product, the upper limit is preferably 100°C, more preferably 60°C, and particularly preferably 30°C. Further, the reaction temperature is preferably not higher than 0°C and particularly not higher than -5°C. The lower limit is preferably -80°C, more preferably -60°C, and particularly preferably -40°C. In particular, if the reaction is conducted at a low temperature of not higher than 0°C, the decomposition of (E)-1,4-dibromo-2-butene is inhibited, and therefore, the 1-arylimino-2-vinylcyclopropanecarboxylic acid derivative (2) can be produced at a high yield.

The reaction time of this reaction is not particularly limited, and may be set as appropriate. The upper limit is preferably 120 hours, more preferably 100 hours, and particularly preferably 80 hours. The lower limit is preferably 0.1 hour, more preferably 1 hour, and particularly preferably 3 hours.

The order in which the compound (1), (E)-1,4-dibromo-2-butene, sodium ethoxide, and the solvent are mixed is not particularly limited. It is preferable to add the compound (1) to a mixture solution of (E)-1,4-dibromo-2-butene and the solvent. It is more preferable to add (E)-1,4-dibromo-2-butene, the solvent, sodium ethoxide, and the compound (1) in this order.

As a treatment after the reaction, a commonly used treatment for obtaining a product from reaction liquid may be conducted. For example, the reaction liquid after the end of the reaction is subjected to an extraction operation using water, or a commonly used extraction solvent, such as ethyl acetate, diethyl ether, methylene chloride, toluene, hexane, or the like. If the reaction solvent and the extraction solvent are distilled away from the extraction liquid using an operation such as heating under reduced pressure or the like, the target material is obtained.

The target material thus obtained has sufficient purity to be used in a subsequent step. In order to further increase the purity, a commonly used purification technique, such as crystallization, fractional distillation, washing by dissolution, column chromatography, or the like, may be used. Preferably, the resultant compound (2) may be used in the following step without being isolated.

In the present invention, after the production of the 1-arylimino-2-vinylcyclopropalecarboxylic acid derivative (2) by the above reaction, a 1-amino-2-vinylcyclopropanecarboxylic acid derivative represented by the following formula (3): 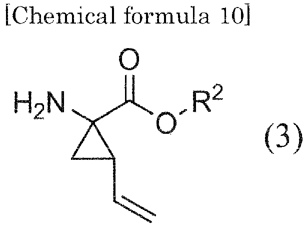 is then preferably produced by acid hydrolysis of the compound (2). Here, R² is the same as R¹ or a hydrogen atom. Specifically, R² represents a C1-C6 alkyl group or a hydrogen atom. More specifically, for example, R² represents a methyl group, an ethyl group, an n-propyl group, an isopropyl group, an n-butyl group, an isobutyl group, a sec-butyl group, a tert-butyl group, or a hydrogen atom, preferably a methyl group, an ethyl group, a tert-butyl group, or a hydrogen atom, and more preferably an ethyl group.

Examples of an acid used in the hydrolysis include inorganic acids such as hydrochloric acid, hydrobromic acid, sulfuric acid, phosphoric acid, nitric acid, perchloric acid, and the like; aromatic sulfonic acids such as p-toluenesulfonic acid, benzenesulfonic acid, and the like; aliphatic sulfonic acids such as methanesulfonic acid, trifluoromethanesulfonic acid, and the like; aliphatic carboxylic acids such as acetic acid, propionic acid, citric acid, malic acid, succinic acid, lactic acid, maleic acid, fumaric acid, and the like; and aromatic carboxylic acids such as phthalic acid, benzoic acid, 4-nitrobenzoic acid, 4-chlorobenzoic acid, and the like. The acids may be used alone or in combination. The acid is preferably an inorganic acid such as hydrochloric acid, hydrobromic acid, sulfuric acid, phosphoric acid, nitric acid, perchloric acid, or the like, more preferably hydrochloric acid.

The amount of the acid to be used is preferably 0.5- 100 equivalents, more preferably 1-30 equivalents, and particularly preferably 2-10 equivalents, with respect to the compound (2). Alternatively, the acid may be successively added to adjust the pH of the mixture of the compound (2) and water within the range of 0-4.

The upper limit of the amount of water required for the acid hydrolysis is preferably 100 times, more preferably 50 times, and particularly preferably 20 times the weight of the compound (2), because the excessive amount is undesirable from a post-treatment standpoint. The lower limit is preferably 0.1 times, more preferably 0.5 times, and particularly preferably one time the weight of the compound (2).

Also, an organic solvent may be further added for the acid hydrolysis in order to ensure the flowability or increase the reaction rate. The organic solvent is not particularly limited as long as it does not have an influence on the acid hydrolysis. Specific examples of the organic solvent include: alcohol solvents such as methanol, ethanol, n-propanol, isopropanol, n-butanol, tert-butanol, ethylene glycol, and the like; ether solvents such as tetrahydrofuran, diethyl ether, 1,4-dioxane, methyl tert-butyl ether, ethylene glycol dimethyl ether, and the like; nitrile solvents such as acetonitrile, propionitrile, and the like; ester solvents such as ethyl acetate, n-propyl acetate, isopropyl acetate, and the like; aliphatic hydrocarbon solvents such as pentane, hexane, heptane, methylcyclohexane, and the like; aromatic hydrocarbon solvents such as benzene, toluene, xylene, ethylbenzene, mesitylene, and the like; ketone solvents such as acetone, methyl ethyl ketone, and the like; halogen solvents such as methylene chloride, 1,2-dichloroethane, chlorobenzene, and the like; sulfoxide solvents such as dimethyl sulfoxide and the like; amide solvents such as N,N-dimethylformamide, N,N-dimethylacetamide, N,N-diethylacetamide, N-methyl-2-pyrrolidone, N-ethyl-2-pyrrolidone. N-methyl-ε-caprolactam, hexamethylphosphoramide, and the like; urea solvents such as dimethylpropyleneurea and the like; phosphoric triamide solvents such as hexamethylphosphoric triamide and the like; and the like. These solvents may be used alone or in combination. When two or more solvents are used in combination, the mixing ratio is not particularly limited. The solvent is preferably ethanol, methyl tert-butyl ether, hexane, heptane, toluene, xylene, ethylbenzene, mesitylene, methylene chloride, 1,2-dichloroethane, or chlorobenzene, more preferably methyl tert-butyl ether or toluene.

The upper limit of the amount of the solvent to be used is preferably 100 times, more preferably 50 times, and particularly preferably 20 times the weight of the compound (2), because the excessive amount is undesirable from a cost or post-treatment standpoint. The lower limit is preferably 0.1 times, more preferably 0.5 times, and particularly preferably one time the weight of the compound (2).

The temperature of the acid hydrolysis is not particularly limited, and may be set as appropriate. In order to reduce the formation of a by-product, the upper limit is preferably 120°C, more preferably 100°C. The lower limit is preferably -20°C and more preferably 0°C. The reaction time of the acid hydrolysis is not particularly limited, and may be set as appropriate. The upper limit is preferably 120 hours, more preferably 100 hours, and particularly preferably 80 hours. The lower limit is preferably 0.1 hour, more preferably 1 hour, and particularly preferably 3 hours.

The order in which the compound (2), the water, the acid, and the organic solvent are mixed is not particularly limited.

As a treatment after the reaction, a commonly used treatment for obtaining a product from reaction liquid may be conducted. For example, the reaction liquid after the end of the reaction is neutralized by adding thereto an aqueous sodium hydroxide solution, an aqueous potassium carbonate solution, or an aqueous sodium hydrogencarbonate solution, and is then subjected to an extraction operation using a commonly used extraction solvent, such as ethyl acetate, diethyl ether, methylene chloride, toluene, hexane, or the like. If the reaction solvent and the extraction solvent are distilled away from the extraction liquid using an operation such as heating under reduced pressure or the like, the target material is obtained.

The compound (3) thus obtained has sufficient purity to be used in a subsequent step. In order to further increase the chemical purity, a commonly used purification technique, such as column chromatography or the like, may be used.

Also, in the present invention, the compound (2) may be subjected to asymmetric hydrolysis to produce a compound represented by the following formula (5): 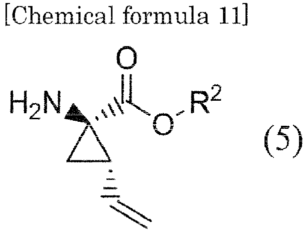 wherein R² is the same as described above. The asymmetric hydrolysis includes hydrolysis using an enzyme, or hydrolysis using an optically active acid or base. Examples of the optically active acid include tartaric acid-based materials such as tartaric acid, tartaric acid esters, tartaric acid amides, and the like, preferably tartaric acid monoarylamides, and particularly tartranilic acid. These tartaric acid-based materials may have substituents as appropriate. For example, a substituent such as a halogen group or the like may be bonded to the phenyl group of tartranilic acid.

The present invention also includes a method of producing a compound, comprising reacting an N-(arylmethylene)glycine ester represented by the following formula (1): 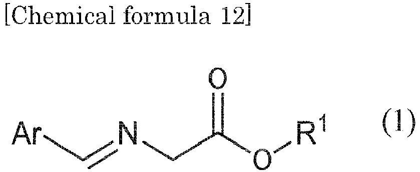 with (E)-1,4-dibromo-2-butene using sodium ethoxide in the presence of an optically active catalyst, preferably an optically active phase-transfer catalyst, to produce the compound represented by the following formula (4): 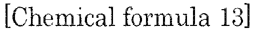 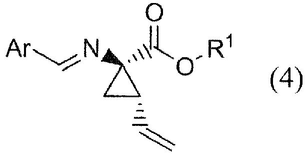 wherein Ar and R¹ are the same as described above. The use of sodium ethoxide allows for asymmetric synthesis.

The optically active phase-transfer catalyst is preferably an optically active quaternary ammonium salt having a biphenyl backbone and/or a binaphtyl backbone, i.e., a Maruoka catalyst (registered trademark), more preferably (11bS)-(+)-4,4-dibutyl-4,5-dihydro-2,6-bis(3,4,5-trifluorophenyl)-3H-dinaphtho[2,1-c:1',2'-elazepinium bromide, (R,R)-3,4,5-trifluorophenyl-NAS bromide, (R,R)-ß-naphtyl-NAS bromide, or (15bS)-14, 14-dibutyl-5,6,7,8,14,15-hexahydro-1,12-bis(3,4,5-trinuorophenyl)-13H-[1,6]benzodioxecino[9.8,7-def][2]benzazepinium bromide, and particularly preferably (11bS)-(+)-4,4-dibutyl-4,5-dihydro-2,6-bis(3,4,5-trifluorophenyl)-3H-dinaphtho[2,1-c: 1',2'-e]azepinium bromide, or (15bS)-14,14-dibutyl-5,6,7,8,14,15-hexahydro-1,12-bis(3,4,5-trifluorophenyl)-13H-[1.6]benzodioxecino[9.8,7-def][2]benzazepinium bromide. The optical purity of the optically active phase-transfer catalyst is not particularly limited. In order to obtain the compound (4) having a high optical purity, the optical purity of the optically active phase-transfer catalyst is preferably not lower than 90% e.e., more preferably not lower than 95% e.e., and particularly preferably not lower than 98% e.e.

The upper limit of the amount of the optically active phase-transfer catalyst to be used is preferably 0.01 equivalents, more preferably 0.005 equivalents, and particularly preferably 0.001 equivalents, with respect to (E)-1,4-dibromo-2-butene, because the excessive amount is undesirable from a cost standpoint. The lower limit is preferably 0.00001 equivalents and more preferably 0.0001 equivalents with respect to (E)-1,4-dibromo-2-butene. Note that, in this step, the amounts of the compound (1), (E)-1,4-dibromo-2-butene, and sodium ethoxide to be used, the reaction temperature, the reaction time, the order in which the reagents are added, and the treatment after the reaction are the same as those for the production of the compound (2) described above.

In the present invention, after the (1R,2S)-1-arylimino-2-vinylcyclopropanecarboxylic acid derivative (4) has been produced using the above reaction, the compound (4) can then be subjected to acid hydrolysis to produce a (1R,2S)-1-amino-2-vinylcyclopropanecarboxylic acid derivative represented by the following formula (5). 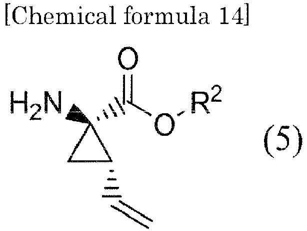 The compound (5) is produced by the same method as that of producing the compound (3) by acid hydrolysis of the compound (2).

This application claims the benefit of priority to Japanese Patent Application No. 2014-68837 filed on March 28, 2014, the entire disclosure of which is incorporated by reference herein.

### EXAMPLE

The present invention will now be described in greater detail by way of examples, which are in no way intended to limit the present invention.

### (Reference Example 1) Production of N-(phenylmethylene)glycine ethyl ester

Glycine ethyl ester hydrochloride (234g (1.68 mol)) and 815 g of toluene were mixed, followed by addition of 170 g (1.60 mol) of benzaldehyde at room temperature. The temperature of the resultant mixture was adjusted to 20°C, and 178 g (1.76 mol) of triethylamine was dropped into the mixture. After the end of the dropping, the mixture was stirred at 20°C for 10 hours. After the end of reaction, 475 g of water was added, followed by stirring for 15 min. The stirring was ended, followed by separation, and the resultant organic layer was subjected to reduced-pressure distillation to obtain 648 g of a toluene solution of N-(phenylmethylene)glycine ethyl ester (the pure content of N-(phenylmethylene)glycine ethyl ester: 294 g (1.54 mmol), yield: 96%).

### (Reference Example 2) Production of N-[(p-chlorophenyl)methylene]glycine ethyl ester

Glycine ethyl ester hydrochloride (42g (302 mmol)) and 146 g of toluene were mixed, followed by addition of 40 g (287 mmol) of p-chlorobenzaldehyde at room temperature. The temperature of the resultant mixture was adjusted to 20°C, and 32 g (316 mmol) of triethylamine was dropped into the mixture. After the end of the dropping, the mixture was stirred at 20°C for 10 hours. After the end of reaction, 85 g of water was added, followed by stirring for 15 min. The stirring was ended, followed by separation, and the resultant organic layer was subjected to reduced-pressure distillation to obtain 126 g of a toluene solution of N-[(p-chlorophenyl)methylene]glycine ethyl ester (the pure content of N-[(p-chlorophenyl)methylene]glycine ethyl ester: 63 g (278 mmol), yield; 97%).

### (Reference Example 3) Production of N-[(p-methoxyphenyl)methylene]glycine ethyl ester

Glycine ethyl ester hydrochloride (18g (126 mmol)) and 61 g of toluene were mixed, followed by addition of 16 g (120 mmol) of p-anisaldehyde at room temperature. The temperature of the resultant mixture was adjusted to 20°C, and 13 g (132 mmol) of triethylamine was dropped into the mixture. After the end of the dropping, the mixture was stirred at 20°C for 10 hours. After the end of reaction, 36 g of water was added, followed by stirring for 15 min. The stirring was ended, followed by separation, and the resultant organic layer was subjected to reduced-pressure distillation to obtain 56 g of a toluene solution of N-[(p-methoxyphenyl)methylene]glycine ethyl ester (the pure content of N-[(p-methoxyphenyl)methylene]glycine ethyl ester: 25 g (114 mmol), yield: 95%).

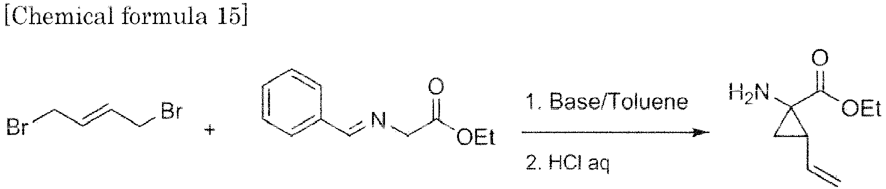

### (Example 1) Production of racemate of 1-amino-2-vinylcyclopropanecarboxylic acid ethyl ester

(E)-1,4-dibromo-2-butene (40g (187 mmol)), 27 g (397 mmol) of sodium ethoxide, and 200 g of toluene were mixed, followed by adjustment of the temperature to -20°C. To the resultant mixture, 95 g of the toluene solution of N-(phenylmethylene)glycine ethyl ester obtained in Reference Example 1 (the pure content of N-(phenylmethylene) glycine ethyl ester: 43 g (225 mmol)) was dropped at -20°C in 5 hours, followed by reaction at -20°C for 6 hours. After the reaction, the temperature of the reaction liquid was adjusted to 0°C, followed by addition of 80 g of water. Next, 24 g of a 35% aqueous hydrochloric acid solution (the pure content of hydrochloric acid: 8 g (230 mmol)) was dropped in 1 hour, followed by stirring for 3 hours. The stirring was ended, followed by separation, and the aqueous layer was removed. To the resultant organic layer, 80 g of water was added, followed by stirring for 30 min. The stirring was ended, followed by separation. As a result, 269 g of an aqueous solution of a racemate of 1-amino-2-vinylcyclopropanecarboxylic acid ethyl ester comprising the resultant aqueous layer (the pure content of the racemate of 1-amino-2-vinylcyclopropanecarboxylic acid ethyl ester: 25 g (163 mmol)) was obtained (yield: 87%). Note that the pure content and the yield were determined by high-performance liquid chromatography analysis under the following conditions.

### [Conditions for high-performance liquid chromatography analysis]

Column: COSMOSIL (registered trademark) 5C8AR-II (4.6 × 250 mm), manufactured by Nacalai Tesque, Inc.
Mobile phrase: mixture solution of the following A- and B-iiquids by gradient pump
A-liquid: 0.1% aqueous phosphoric acid solution
B-liquid: acetonitrile
B-liquid concentration (vol%) in mobile phase: 5% (5 min) → 40% (25 min) → 80% (40 min) → 80% (45 min) → 5% (45.1 min) → 5% (55 min)
Flow rate: 1.0 ml/min
Detector wavelength: 210 nm
Retention time: 9.3 min (1-amino-2-vinylcyclopropanecarboxylic acid ethyl ester)

### (Comparative Examples 1-10) Production of racemate of 1-amino-2-vinylcyclopropanecarboxylic acid ethyl ester

A reaction between N-(phenylmethylene)glycine ethyl ester and (E)-1,4-dibromo-2-butene was conducted by performing an operation similar to that of Example 1 described above using bases described below to obtain racemates of 1-amino-2-vinylcyclopropanecarboxylic acid ethyl ester. The resultant aqueous solutions were analyzed under the conditions described in Example 1 to calculate the yields of the racemates of 1-amino-2-vinylcyclopropanecarboxylic acid ethyl ester. The results are shown in Table 1.

**[Table 1]**

| Run | Base | Yield |
|---|---|---|
| Example 1 | NaOEt | 87% |
| Comparative Example 1 | LiOH | 0% |
| Comparative Example 2 | LiOEt | 48% |
| Comparative Example 3 | LiOₜBu | 84% |
| Comparative Example 4 | NaOH | 42% |
| Comparative Example 5 | NaOMe | 24% |
| Comparative Example 6 | NaOⁱPr | 33% |
| Comparative Example 7 | NaO^{t}Bu | 53% |
| Comparative Example 8 | KOH | 43% |
| Comparative Example 9 | KOEt | 0% |
| Comparative Example 10 | KO^{t}Bu | 0% |

### (Example 2) Production of racemate of 1-amino-2-vinylcyclopropanecarboxylic acid ethyl ester

(E)-1,4-dibromo-2-butene (8.4g (39 mmol)), 5.6 g (82 mmol)) of sodium ethoxide, and 42 g of toluene were mixed, followed by adjustment of the temperature to -20°C. To the resultant mixture, 21.2 g of the toluene solution of N-[(p-chlorophenyl)methylene]glycine ethyl ester obtained in Reference Example 2 (the pure content of N-[(p-chlorophenyl)methyleneglycine ethyl ester: 10.6 g (47 mmol)) was dropped at -20°C in 2 hours, followed by reaction at -20°C for 2 hours. After the end of the reaction, the temperature of the reaction liquid was adjusted to 0°C, followed by addition of 17 g of water. Next, 4.5 g of a 35% aqueous hydrochloric acid solution (the pure content of hydrochloric acid: 1.6 g (43 mmol)) was dropped in 1 hour, followed by stirring for 1 hour. The stirring was ended, followed by separation, and the aqueous layer was removed. To the resultant organic layer, 17 g of water was added, followed by stirring for 30 min. The stirring was ended, followed by separation. As a result, 56 g of an aqueous solution of a racemates of 1-amino-2-vinylcyclopropanecarboxylic acid ethyl ester comprising the resultant aqueous layer (the pure content of the racemate of 1-amino-2-vinylcyclopropanecarboxylic acid ethyl ester: 4.5 g (29 mmol)). The resultant aqueous solution was analyzed under the conditions described in Example 1 to calculate the yield of the racemate of 1-amino-2-vinylcyclopropanecarboxylic acid ethyl ester (yield: 74%).

### (Example 3) Production of racemate of 1-amino-2-vinylcyclopropanecarboxylic acid ethyl ester

(E)-1,4-dibromo-2-butene (8.1g (38 mmol)), 5.4 g (80 mmol) of sodium ethoxide, and 41 g of toluene were mixed, followed by adjustment of the temperature to -20°C. To the resultant mixture, 22.5 g of the toluene solution of N-[(p-methoxyphenyl)methylene]glycine ethyl ester obtained in Reference Example 3 (the pure content of N-[(p-methoxyphenyl)methylene]glycine ethyl ester: 10.1 g (46 mmol)) was dropped at -20°C in 2 hours, followed by reaction at -20°C for 2 hours. After the end of the reaction, the temperature of the reaction liquid was adjusted to 0°C, followed by addition of 16 g of water. Next, 4.4 g of a 35% aqueous hydrochloric acid solution (the pure content of hydrochloric acid: 1.5 g (42 mmol)) was dropped in 1 hour, followed by stirring for 1 hour. The stirring was ended, followed by separation, and the aqueous layer was removed. To the resultant organic layer, 16 g of water was added, followed by stirring for 30 min. The stirring was ended, followed by separation. As a result, 55 g of an aqueous solution of a racemate of 1-amino-2-vinylcyclepropanecarboxylic acid ethyl ester comprising the resultant aqueous layer (the pure content of the racemate of 1-amino-2-vinylcyclopropanecarboxylic acid ethyl ester: 4.5 g (29 mmol)). The resultant aqueous solution was analyzed under the conditions described in Example 1 to calculate the yield of the racemate of 1-amino-2-vinylcyclopropanecarboxylic acid ethyl ester (yield: 76%).

### (Example 4) Production of racemate of 1-amino-2-vinylcyclopropanecarboxylic acid ethyl ester

(E)-1,4-dibromo-2-butene (8.3g (39 mmol)), 5.6 g (82 mmol) of sodium ethoxide, and 42 g of methyl tert-butyl ether were mixed, followed by adjustment of the temperature to -20°C. To the resultant mixture, 19.8 g of the toluene solution of N-(phenylmethylene)glycine ethyl ester obtained in Reference Example 1 (the pure content of N-(phenylmethylene)glycine ethyl ester: 8.9 g (47 mmol)) was dropped at -20°C in 2 hours, followed by reaction at -20°C for 2 hours. After the end of the reaction, the temperature of the reaction liquid was adjusted to 0°C, followed by addition of 17 g of water. Next, 4.5 g of a 35% aqueous hydrochloric acid solution (the pure content of hydrochloric acid: 1.6 g (43 mmol)) was dropped in 1 hour, followed by stirring for 1 hour. The stirring was ended, followed by separation, and the aqueous layer was removed. To the resultant organic layer, 17 g of water was added, followed by stirring for 30 min. The stirring was ended, followed by separation. As a result, 57 g of an aqueous solution of a racemate of 1-amino-2-vinylcyclopropanecarboxylic acid ethyl ester comprising the resultant aqueous layer (the pure content of the racemate of 1-amino-2-vinylcyclopropanecarboxylic acid ethyl ester: 4.6 g (30 mmol)). The resultant aqueous solution was analyzed under the conditions described in Example 1 to calculate the yield of the racemate of 1-amino-2-vinylcyclopropanecarboxylic acid ethyl ester (yield: 77%).

### (Example 5) Production of racemate of 1-amino-2-vinylcyclopropanecarboxylic acid ethyl ester

(E)-1,4-dibromo-2-butene (6.4g (30 mmol)), 4.3 g (63 mmol) of sodium ethoxide, and 32 g of dichloromethane were mixed, followed by adjustment of the temperature to -20°C. To the resultant mixture, 15.3 g of the toluene solution of N-(phenylmethylene)glycine ethyl ester obtained in Reference Example 1 (the pure content of N-(phenylmethylene) glycine ethyl ester: 6.9 g (36 mmol)) was dropped at -20°C in 2 hours, followed by reaction at -20°C for 2 hours. After the end of the reaction, the temperature of the reaction liquid was adjusted to 0°C, followed by addition of 13 g of water. Next, 3.4 g of a 35% aqueous hydrochloric acid solution (the pure content of hydrochloric acids: 1.2 g (33 mmol)) was dropped in 1 hour, followed by stirring for 1 hour. The stirring was ended, followed by separation, and the aqueous layer was removed. To the resultant organic layer, 13 g of water was added, followed by stirring for 30 min. The stirring was ended, followed by separation. As a result, 66.6 g of an aqueous solution of a racemate of 1-amino-2-vinylcyclopropanecarboxylic acid ethyl ester comprising the resultant aqueous layer (the pure content of the racemate of 1-amino-2-vinylcyclopropanecarboxylic acid ethyl ester: 3.3 g (22 mmol)). The resultant aqueous solution was analyzed under the conditions described in Example 1 to calculate the yield of the racemate of 1-amino-2-vinylcyclopropanecarboxylic acid ethyl ester (yield: 73%).

### (Example 6) Production of (1R,2S)-1-amino-2-vinylcyclopropanecarboxylic acid ethyl ester

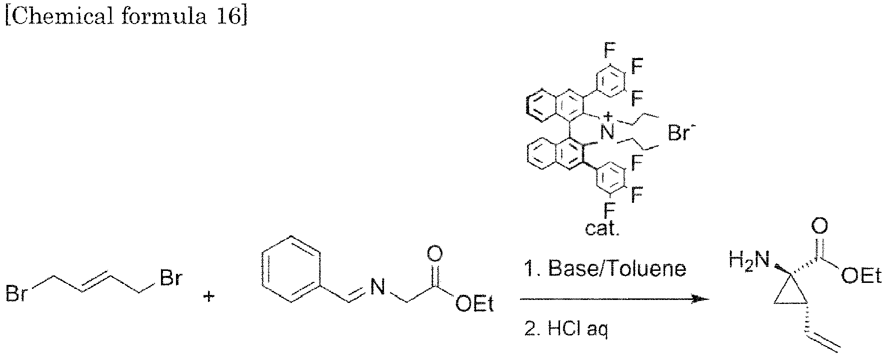

(11bS)-(+)-4,4-dibutyl-4,5-dihydro-2,6-bis(3,4,5-trifluorophenyl)-3H-dinaphtho[2,1-c: 1',2'-e]azepinium bromide (108mg (0.14 mmol)), 6.2 g (29.0 mmol) of (E)-1,4-dibromo-2-butene, 4.1 g (60.8 mmol) of sodium ethoxide, and 31 g of toluene were mixed, followed by adjustment of the temperature to -20°C. To the resultant mixture, 14.6 g of the toluene solution of N-(phenylmethylene)glycine ethyl ester obtained in Reference Example 1 (the pure content of N-(phenylmethylene)glycine ethyl ester: 6.7 g (34.8 mmol)) was dropped at -20°C in 2 hours, followed by reaction at -30°C for 1 hour. After the end of the reaction, the temperature of the reaction liquid was adjusted to 0°C, followed by addition of 12 g of water. Next, 3.6 g of a 35% aqueous hydrochloric acid solution (the pure content of hydrochloric acid: 1.3 g (34.8 mmol)) was dropped in 1 hour, followed by stirring for 3 hours. The stirring was ended, followed by separation, and the aqueous layer was removed. To the resultant organic layer, 12 g of water was added, followed by stirring for 30 min. The stirring was ended, followed by separation. As a result, 41.5 g of an aqueous solution of (1R,2S)-1-amino-2-vinylcyclopropane-1-carboxylic acid ethyl ester comprising the resultant aqueous layer (the pure content of (1R,2S)-1-amino-2-vinylcyclopropanecarboxylic acid ethyl ester: 3.5 g (22.7 mmol)), in which the compound is contained in a larger amount than that of the enantiomer thereof, was obtained (yield: 79%, optical purity: 59% e.e.). The resultant aqueous solution was analyzed under the high-performance liquid chromatography analysis conditions described in Example 1 to calculate the yield of (1R,2S)-1-amino-2-vinylcyclopropanecarboxylic acid ethyl ester. Also, the optical purity was determined by the following analysis.

### [Optical purity analysis]

Column: CHIRALPAK (trade name) AD-RH (4.6 × 150 mm, 5 µm), manufactured by Daicel Corporation
Mobile phase: mixture solution of the following A- and B-liquids where A:B = 6/4 (volume ratio)
A-liquid: methanol
B-liquid: 20 mM aqueous ammonium bicarbonate solution (pH 9.0) A/B = 6/4
Flow rate : 1.0 ml/min
Detector wavelength: 220 nm
Retention time: 4.9 min ((1R,2S)-1-amino-2-vinylcyclopropanecarboxylic acid ethyl ester), 8.4 min ((1S,2R)-1-amino-2-vinylcyclopropanecarboxylic acid ethyl ester)

### (Comparative Example 11) Production of (1R,2S)-1-amino-2-vinylcyclopropanecarboxylic acid ethyl ester

A reaction between N-(phenylmethylene)glycine ethyl ester and (E)-1,4-dibromo-2-butene was conducted by performing an operation similar to that of Example 6 described above using bases described below, in the presence of (11bS)-(+)-4,4-dibutyl-4,5-dihydro-2,6-bis(3,4,5-trifluorophenyl)-3H-dinaphtho[2,1-c: 1',2'-elazepinium bromide, to obtain 1-amino-2-vinylcyclopropanecarboxylic acid ethyl ester. The resultant aqueous solution was analyzed under the high-performance liquid chromatography analysis conditions described in Example 1 to calculate the yield of 1-amino-2-vinylcyclopropanecarboxylic acid ethyl ester. The aqueous solution was also analyzed under the optical purity analysis conditions described in Example 6 to calculate the optical purity of (1R,2S)-1-amino-2-vinylcyclopropanecarboxylic acid ethyl ester. The results are shown in Table 2.

**[Table 2]**

| Run | Base | Yield | Optical purity |
|---|---|---|---|
| Comparative Example 11 | Lio^{t}Bu | 67% | 0% e.e. |
| Example 6 | NaOEt | 79% | 59% e.e. |

### (Example 7) Production of (1R,2S)-1-amino-2-vinylcyclopropanecarboxylic acid ethyl ester

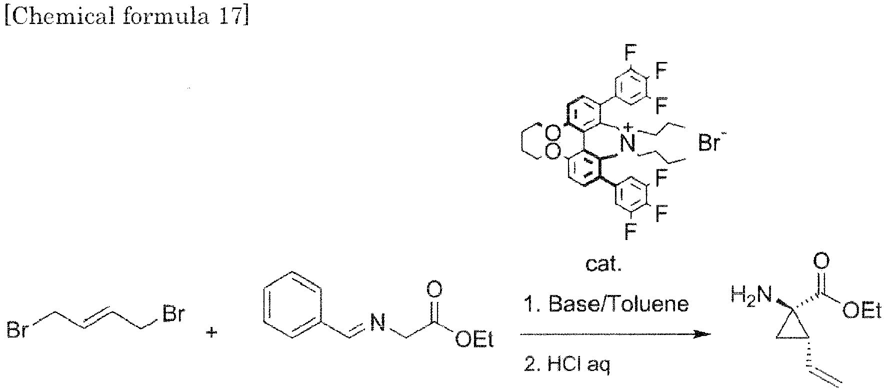

(15bS)-14,14-dibutyl-5,6,7,8,14,15-hexahydro-1,12-bis(3,4,5-trifluorophenyl)-13H-[1,6]benzodioxecino[9.8,7-def][2]benzazepinium bromide, 0.43 g (2.0 mmol) of (E)-1,4-dibromo-2-butene (7.3mg (0.01 mmol)), 0.29 g (4.2 mmol) of sodium ethoxide, and 2.15 g of toluene were mixed, followed by adjustment of the temperature to -20°C. To the resultant mixture, 1.01 g of the toluene solution of N-(phenylmethylene)glycine ethyl ester obtained in Reference Example 1 (the pure content of N-(phenylmethylene)glycine ethyl ester: 0.46 g (2.4 mmol)) was dropped at -20°C in 2 hours, followed by reaction at -30°C for 1 hour. After the end of the reaction, the temperature of the reaction liquid was adjusted to 0°C, followed by addition of 0.86 g of water. Next, 0.25 g of a 35% aqueous hydrochloric acid solution (the pure content of hydrochloric acid: 0.09 g (2.4 mmol)) was dropped in 1 hour, followed by stirring for 3 hours. The stirring was ended, followed by separation, and the aqueous layer was removed. To the resultant organic layer, 0.86 g of water was added, followed by stirring for 30 min. The stirring was ended, followed by separation. As a result, 2.80 g of an aqueous solution of(1R,2S)-1-amino-2-vinylcyclopropane-1-carboxylic acid ethyl ester comprising the resultant aqueous layer (the pure content of (1R,2S)-1-amino-2-vinylcyclopropanecarboxylic acid ethyl ester: 0.12 g (0.76 mmol)), in which the compound is contained in a larger amount than that of the enantiomer thereof, was obtained. The resultant aqueous solution was analyzed under the high-performance liquid chromatography analysis conditions described in Example 1 to calculate the yield of (1R,2S)-1-amino-2-vinylcyclopropanecarboxylic acid ethyl ester. The aqueous solution was also analyzed under the optical purity analysis conditions described in Example 6 to calculate the optical purity of (1R,2S)-1-amino-2-vinylcyclopropanecarboxylic acid ethyl ester (yield: 38%, optical purity: 39% e.e.).

### (Example 8) Production of (1R,2S)-1-amino-2-vinylcyclopropanecarboxylic acid tert-butyl ester

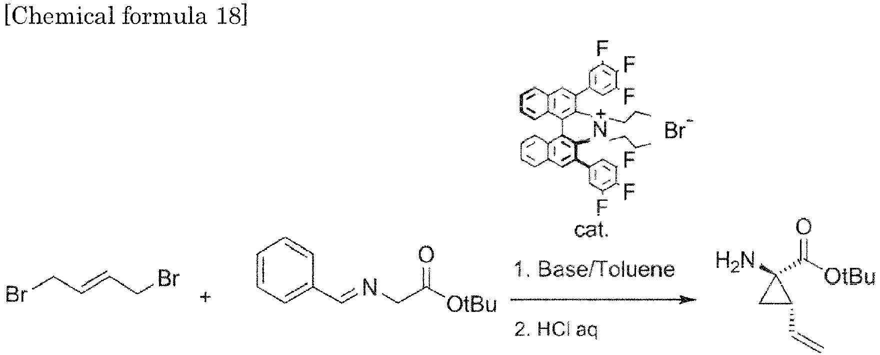

(11bS)-(+)-4,4-dibutyl-4,5-dihydro-2,6-bis(3,4,5-trifluorophenyl)-3H-dinaphtho[2,1-c: 1',2'-e]azepinium bromide (28.1mg (0.04 mmol)), 1.6 g (7.5 mmol) of (E)-1,4-dibromo-2-butene, 1.1 g (15.8 mmol) of sodium ethoxide, and 8.0 g of toluene were mixed, followed by adjustment of the temperature to -20°C. To the resultant mixture, 4.5 g of a toluene solution of N-(phenylmethylene)glycine tert-butyl ester (the pure content of N-(phenylmethylene)glycine tert-butyl ester: 2.0 g (9.0 mmol)) was dropped at -20°C in 2 hours, followed by reaction at -30°C for 1 hour. After the end of the reaction, the temperature of the reaction liquid was adjusted to 0°C, followed by addition of 3.2 g of water. Next, 0.9 g of a 35% aqueous hydrochloric acid solution (the pure content of hydrochloric acid: 0.3 g (9.0 mmol)) was dropped in 1 hour, followed by stirring for 3 hours. The stirring was ended, followed by separation, and the aqueous layer was removed. To the resultant organic layer, 3.2 g of water was added, followed by stirring for 30 min. The stirring was ended, followed by separation. As a result, 12.1 g of an aqueous solution of (1R,2S)-1-amino-2-vinylcyclopropane-1-carboxylic acid tert-butyl ester comprising the resultant aqueous layer (the pure content of (1R,2S)-1-amino-2-vinylcyclopropane-1-carboxylic acid tert-butyl ester: 0.3 g (1.7 mmol)), in which the compound is contained in a larger amount than that of the enantiomer thereof, was obtained. The resultant aqueous solution was analyzed under the high-performance liquid chromatography analysis conditions described in Example 1 to calculate the yield of (1R,2S)-1-amino-2-vinylcyclopropane-1-carboxylic acid tert-butyl ester. The aqueous solution was also analyzed under the optical purity analysis conditions described in Example 6 to calculate the optical purity of (1R,2S)-1-amino-2-vinylcyclopropane-1-carboxylic acid tert-butyl ester (yield: 23%, optical purity: 35% e.e.). Note that the retention time of each optical isomer under the analysis conditions described in Example 6 is as follows.

### [Optical purity analysis]

Retention time: 6.7 min ((1R,2S)-1-amino-2-vinylcyclopropanecarboxylic acid tert-butyl ester), 14.5 min ((1S,2R)-1-amino-2-vinylcyclopropanecarboxylic acid tert-butyl ester)

### INDUSTRIAL APPLICABILITY

The method of the present invention is applicable to the production of a (1R,2S)-1-amino-2-vinylcyclopropanecarboxylic acid derivative useful as a pharmaceutical intermediate, such as, for example, an intermediate for a therapeutic agent for hepatitis C.

## Claims

1. A method of producing a 1-arylimino-2-vinylcyclopropanecarboxylic acid derivative, comprising
reacting an N-(arylmethylene)glycine ester represented by the following formula (1): 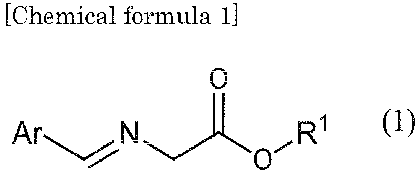 whereinAr represents a C₆-C₁₂ aryl group and R¹ represents a C₁-C₆ alkyl group, with (E)-1,4-dibromo-2-butene using a sodium ethoxide, to produce the 1-arylimino-2-vinylcyclopropanecarboxylic acid derivative represented by the following formula (2) : 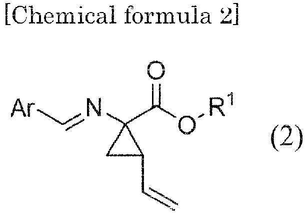 wherein Ar and R¹ are the same as described above.

2. A method of producing a 1-amino-2-vinylcyclopropanecarboxylic acid derivative, comprising
producing a 1-arylimino-2-vinylcyclopropanecarboxylic acid derivative (2) by the reaction as recited in claim 1,
undergoing acid hydrolysis successively, to produce the 1-amino-2-vinylcyclopropanecarboxylic acid derivative represented by the following formula (3): 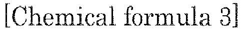 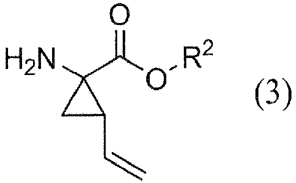 wherein R² represents a C₁-C₆ alkyl group or a hydrogen atom.

3. The method according to claim 1 or 2, wherein
R¹ is a methyl group, an ethyl group, an isopropyl group, an n-butyl group, an isobutyl group, a sec-butyl group, or a tert-butyl group, and
R² is a methyl group, an ethyl group, an isopropyl group, an n-butyl group, an isobutyl group, a sec-butyl group, a tert-butyl group, or a hydrogen atom.

4. The method according to any one of claims 1 to 3, wherein
Ar is a phenyl group, a p-chlorophenyl group, or a p-methoxyphenyl group.

5. The method according to any one of claims 1 to 4, wherein
R¹ is an ethyl group, R² is an ethyl group, and Ar is a phenyl group.

6. A method of producing a (1R,2S)-1-arylimino-2-vinylcyclopropanecarboxylic acid derivative, comprising
reacting an N-(arylmethylene)glycine ester represented by the following formula (1): 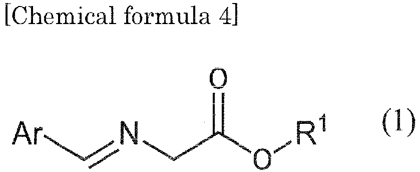 whereinAr represents a C₆-C₁₂ aryl group, and R¹ represents a C₁-C₆ alkyl group, with (E)-1,4-dibromo-2-butene using a sodium ethoxide in the presence of an optically active catalyst, to produce the (1R,2S)-1-arylimino-2-vinylcyclopropanecarboxylic acid derivative represented by the following formula (4): 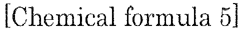 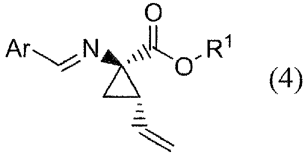 wherein Ar and R¹ are the same as described above.

7. A method of producing a (1R,2S)-1-amino-2-vinylcyclopropanecarboxylic acid derivative, comprising
producing a (1R,2S)-1-arylimino-2-vinylcyclopropanecarboxylic acid derivative (4) by the reaction as recited in claim 6,
undergoing acid hydrolysis successively to produce the (1R,2S)-1-amino-2-vinylcyclopropanecarboxylic acid derivative represented by the following formula (5): 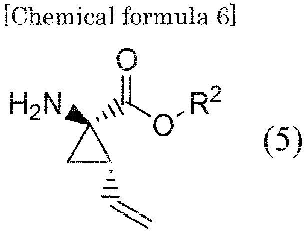 wherein R² represents a C₁-C₆ alkyl group or a hydrogen atom.

8. The method according to any one of claims 1 to 7, wherein
the reaction is conducted at a temperature of not higher than 0°C.

9. The method according to claim 6, wherein
the optically active catalyst is a phase-transfer catalyst.

10. The method according to claim 9, wherein
the phase-transfer catalyst is (11bS)-(+)-4,4-dibutyl-4,5-dihydro-2,6-bis(3,4,5-trifluorophenyl)-3H-dinaphtho[2,1-c:1',2'-e]azepinium bromide, or (15bS)-14,14-dibutyl-5,6,7,8,14,15-hexahydro-1,12-bis(3,4,5-trifluorophenyl)-13H-[1,6]benzodioxecino[9.8,7-def][2]benzazepinium bromide.

11. The method according to any one of claims 7 to 10, wherein
R¹ is a methyl group, an ethyl group, an isopropyl group, an n-butyl group, an isobutyl group, a sec-butyl group, or a tert-butyl group, and
R² is a methyl group, an ethyl group, an isopropyl group, an n-butyl group, an isobutyl group, a sec-butyl group, a tert-butyl group, or a hydrogen atom.

12. The method according to any one of claims 7 to 11, wherein
Ar is a phenyl group, a p-chlorophenyl group, or a p-methoxyphenyl group.

13. The method according to any one of claims 6 to 12, wherein
R¹ is an ethyl group, R² is an ethyl group, and Ar is a phenyl group.

14. The method according to any one of claims 6 to 13, wherein
the amount of the optically active catalyst to be used is not more than 0.01 equivalents with respect to (E)-1,4-dibromo-2-butene.
